# EUROPEAN PATENT APPLICATION

(11) **EP 2 351 558 A1**
(43) Date of publication of application: **03.08.2011**
(21) Application number: 09816247.2
(22) Date of filing: 28.09.2009
(51) Int. Cl.: A61K 31/05, A23K 1/16, A23K 1/18, A61K 31/60, A61K 36/18, A61P 1/00

(54) **THERAPEUTIC AGENT FOR TYMPANITES IN RUMINANT ANIMAL**

(30) Priority: 29.09.2008 JP 2008250241
(71) Applicant: Idemitsu Kosan Co., Ltd., Chiyoda-ku Tokyo 100-8321 (JP)
(72) Inventor: SUZUKI, Chie, Tokyo 130-0015 (JP); NAGASHIMA, Kyo, Sodegaura-shi Chiba 299-0293 (JP); MOCHIZUKI, Masami, Sodegaura-shi Chiba 299-0293 (JP)
(74) Representative: Vossius & Partner
(86) International application number: PCT/JP2009/066788
(87) International publication number: WO 2010/035833

(57) **Abstract**

The present invention provides a bloat therapeutic agent for a ruminant comprising cashew nut shell liquid, heated cashew nut shell liquid, anacardic acid, cardanol, or anacardic acid and cardanol.

## Description

### Technical Field

The present invention relates to a bloat therapeutic agent comprising cashew nut shell liquid, heated cashew nut shell liquid, anacardic acid, cardanol, or anacardic acid and cardanol.

### Background Art

Bloat is a disease which occurs in a ruminant such as a bovine or a sheep, and it is considered that one of the reasons therefor is that it becomes difficult to discharge gas generated by rumen fermentation. The ruminant suffering from bloat has symptoms of abnormal hypertrophy of rumen capacity, decline in feeding activity, decline in rumination behavior, dyspnea, and the like, which are caused by accumulation of fermentation gas, and may die in the case where those symptoms are serious. In addition, there are known acute bloat and chronic bloat. In the case of the acute bloat, progression of the symptom is extremely rapid, and the ruminant dies of dyspnea in many cases. On the other hand, in the case of the chronic bloat, the generation of fermentation gas becomes chronic, so the therapeutic practice therefore needs to be repeated many times. Therefore, there are caused decrease in productivity of domestic animals and exhaustion of labor for the therapeutic practice. Because of those symptoms, the bloat is recognized as one of the major diseases that causes large economic loss in production field of domestic animals.
Researches on factors for occurrence of bloat are under progress by various research institutes, but the factors has not become clear yet. From the experimental knowledge, it is considered that the occurrence of bloat is related with a saliva composition (insufficient secretion of mucin), a grain size of feed, a ratio of coarse feed, a supply amount of leguminous feed, a supply amount of easily fermentable feed (cereals such as barley), an addition amount of salt, and the like. Further, it is also considered as a major reason for the occurrence of bloat that *Streptococcus bovis,* which is an indigenous bacterium of the rumen and is a gram-positive bacterium, is formed into a capsule and accumulates polysaccharides outside the cell, to thereby increase the viscosity of rumen juice and inhibit the discharge of fermentation gas through a foam. However, it may be difficult to adjust the above conditions based on a nutritional feed design, a difference between individual animal, and the like.
Consequently, it is difficult to control the occurrence of bloat and the economic loss caused by the occurrence of bloat is tremendous, and hence, the needs for an effective therapeutic method and an effective therapeutic agent are extremely high.

As conventional main therapy for bloat, there have been performed degassing of fermentation gas, use of an anti-foaming agent, and administration of a gastrointestinal agent for enhancing the peristaltic movement of gastrointestinal tracts. Ideally, both of palliative therapies with urgency (degassing and defoaming) and radical therapies (improvement in properties of rumen juice and sterilization of *Streptococcus bovis*) are necessary. Conventionally, a technology/product that satisfies both of the above by a single agent has not been generalized. Physical palliative therapies (degassing, defoaming, and utilization of rumination promoting material) are each a temporary therapy and do not solve an underlying cause, and hence, it is not considered that the bloat is cured. In particular, in the case of the above-mentioned chronic bloat, fermentation gas tends to be generated and accumulated, and hence, the effect exhibited by those palliative therapies is small. In addition, since the degassing includes an operation of inserting an injection needle into integument to thereby degas or inserting a tube into oral cavity or nasal cavity to thereby degas, the degassing requires a great deal of labor and is complicated, and also, the stress on animals is heavy. Further, an antibiotic having a sterilizing effect against *Streptococcus bovis* is present, but the practical application thereof as an effective therapeutic agent has not been realized.
It is known that cashew nut shell liquid has an antibacterial action (Non-patent Documnet 1) and a coccidiosis alleviating action (Patent Document 1), but there are no findings at all on the therapeutic effect on bloat of a ruminant.

### Prior Art Documents

### Patent Document

Patent Document 1: JP 08-231410 A

### Non-patent Document

Non-patent Document 1: Muroi, H. et al. Bioorganic & Medicinal Chemistry 12, 583-587 (2004)

### Disclosure of the Invention

An object of the present invention is to control bloat of a ruminant.

The inventors of the present invention have conducted intensive studies in order to solve the above problems, and as a result, they have found that cashew nut shell liquid, heated cashew nut shell liquid, anacardic acid, cardanol, or anacardic acid and cardanol can be used for the bloat therapy. Thus, the inventors of the present invention have completed the present invention.

That is, the summary of the present invention is as follows.
(1) A bloat therapeutic agent comprising cashew nut shell liquid, heated cashew nut shell liquid, anacardic acid, cardanol, or anacardic acid and cardanol.
(2) A composition for a feed for bloat therapy, comprising cashew nut shell liquid, heated cashew nut shell liquid, anacardic acid, cardanol, or anacardic acid and cardanol.
(3) A feed for bloat therapy, comprising cashew nut shell liquid, heated cashew nut shell liquid, anacardic acid, cardanol, or anacardic acid and cardanol.
(4) The bloat therapeutic agent according to (1), wherein the bloat is caused by *Streptococcus bovis.*
(5) A composition for a feed for bloat therapy, comprising the bloat therapeutic agent according to (1) or (4).
(6) A feed for bloat therapy, comprising the composition for a feed for bloat therapy according to (5).
(7) A method for bloat therapy, comprising administering cashew nut shell liquid, heated cashew nut shell liquid, anacardic acid, cardanol, or anacardic acid and cardanol to a ruminant suffering from bloat.
(8) The method for bloat therapy according to (7), wherein the cashew nut shell liquid, heated cashew nut shell liquid, anacardic acid, cardanol, or anacardic acid and cardanol is administered 1 to 1,000 ml per ruminant per day.
(9) The method for bloat therapy according to (7) or (8), wherein the bloat is caused by *Streptococcus bovis.*
(10) Cashew nut shell liquid, heated cashew nut shell liquid, anacardic acid, cardanol, or anacardic acid and cardanol for use in manufacturing a bloat therapeutic agent.
(11) Cashew nut shell liquid, heated cashew nut shell liquid, anacardic acid, cardanol, or anacardic acid and cardanol for use in manufacturing a composition for a feed for bloat therapy.
(12) Cashew nut shell liquid, heated cashew nut shell liquid, anacardic acid, cardanol, or anacardic acid and cardanol for use in manufacturing a feed for bloat therapy.
(13) Cashew nut shell liquid, heated cashew nut shell liquid, anacardic acid, cardanol, or anacardic acid and cardanol according to any one of (10) to (12), wherein the bloat is caused by *Streptococcus bovis.*

The bloat therapeutic agent of the present invention can control bloat by being administered to a ruminant suffering from bloat. Further, the bloat therapeutic agent of the present invention can also prevent a relapse of bloat. That is, the bloat therapeutic agent of the present invention has both a defoaming effect and a sterilizing/suppressing effect against *S*. *bovis.*

### Best Mode for carrying out the Invention

The bloat therapeutic agent for a ruminant of the present invention includes cashew nut shell liquid, heated cashew nut shell liquid, anacardic acid, cardanol, or anacardic acid and cardanol.

The cashew nut shell liquid to be used in the present invention is an oily liquid contained in the shell of the seed of a cashew nut tree (*Anacardium occidentale* L.). The cashew nut shell liquid contains, as the components thereof, anacardic acid, cardanol, and cardol. Since anacardic acid is a main component for developing the activity, it is preferred that a ratio of anacardic acid is high. In general, anacardic acid is converted to cardanol by heat treatment.
Non-heated cashew nut shell liquid extracted by compressing cashew nut shells contains 55 to 80 mass% of anacardic acid, 5 to 20 mass% of cardanol, and 5 to 30 mass% of cardol as described in J. Agric. Food Chem. 2001, 49, 2548-2551.
Heated cashew nut shell liquid obtained by heat treatment of non-heated cashew nut shell liquid at 130 °C or more, wherein the large part of anacardic acid which is a main component of non-heated cashewnut shell liquid is decarboxylated and converted to cardanol, contains 0 to 10 mass% of anacardic acid, 55 to 80 mass% of cardanol, and 5 to 30 mass% of cardol.

The cashew nut shell liquid used in the present invention can be obtained as a vegetable oil extracted by compressing the shell of a cashew nut. Further, the cashew nut shell liquid used in the present invention can also be obtained by heating or extracting, e.g., dry-distilling or solvent-extracting a cashew nut shell. In addition, the cashew nut shell liquid used in the present invention can be obtained according to a method described in JP 08-231410 A.
The cashew nut shell liquid used in the present invention may also be a liquid obtained by pulverizing/crushing the shell of a cashew nut without heating. Further, the cashew nut shell liquid used in the present invention may also be a shell of a cashew nut itself.
For the cashew nut shell liquid used in the present invention, a commercially-available product may also be used.

The cashew nut shell liquid of the present invention may be heated cashew nut shell liquid obtained by heat treatment of non-heated cashew nut shell liquid, which was obtained as above, at 70°C or more, preferably at 130 °C or more.
The cashew nut shell liquid of the present invention may be obtained by compressing cashew nut shells and extracting cashew nut shell liquid (non-heated), and heating the cashew nut shell liquid at 130 °C.
The bloat therapeutic agent of the present invention can comprise heated cashew nut shell liquid, anacardic acid, cardanol, or anacardic acid and cardanol instead of cashew nut shell liquid.

As anacardic acids used in the present invention, there are exemplified natural anacardic acid, synthetic anacardic acid, and the derivatives thereof. Further, commercially-available anacardic acidmaybe used. As described in JP 08-231410 A, anacardic acids may be obtained, for example, by eluting the cashew nut shell liquid, which has been obtained by subjecting the cashew nut shell to extraction treatment with an organic solvent, through chromatography on a silica gel column using a solvent of n-hexane, ethyl acetate, and acetic acid mixed at varied ratios (JP 03-240721 A, JP03-240716A, and the like). Such anacardic acid can be comprised in a bloat therapeutic agent, a composition for a feed for bloat therapy, and a feed for bloat therapy in the same content as cashew nut shell liquid.

As cardanol used in the present invention, there are exemplified natural cardanol, synthetic cardanol, and the derivatives thereof. Cardanol used in the present invention can be obtained by decarboxylating anacardic acid which is a main component of cashew nut shell liquid.
Meanwhile, when heated cashew nut shell liquid is used, the mass ratio of anacardic acid: cardanol is preferably 0:100 to 20: 80.

The content of the cashew nut shell liquid, heated cashew nut shell liquid, anacardic acid, cardanol, or anacardic acid and cardanol in the bloat therapeutic agent of the present invention is, from the viewpoints of effects and costs, preferably 1 mass% to 100 mass%, more preferably 5 mass% to 100 mass%, and still more preferably 10 mass% to 100 mass%, with respect to a total amount of the bloat therapeutic agent. When the content is 1 mass% or more, the bloat can be controlled effectively.

In the present invention, a stock solution of cashew nut shell liquid may be directly orally administered.
In the case where the stock solution of the cashew nut shell liquid of the present invention is directly administered, the dose of the cashew nut shell liquid is preferably 1 to 1,000 ml per ruminant per day, more preferably 5 to 500 ml per ruminant per day, and most preferably 10 to 300 ml per ruminant per day. Meanwhile, when heated cashew nut shell liquid, anacardic acid, cardanol, or anacardic acid and cardanol is administered, it can be administered in the same dose as cashew nut shell liquid.

The bloat therapeutic agent of the present invention can be used for controlling bloat caused by *Streptococcus bovis.*
The bloat therapeutic agent according to the present invention can also be used for the bloat caused by excessive administration of leguminous pasture or concentrated feed.

The bloat therapeutic agent of the present invention may contain, in addition to the cashew nut shell liquid, heated cashew nut shell liquid, anacardic acid, cardanol, or anacardic acid and cardanol, any excipient as long as it can be used for a feed, a drug, or a food product, such as lactose, saccharose, D-mannitol, starch, corn starch, crystalline cellulose, silica gel, and light anhydrous silicic acid.

The bloat therapeutic agent of the present invention may contain, in addition to the cashew nut shell liquid, heated cashew nut shell liquid, anacardic acid, cardanol, or anacardic acid and cardanol, an arbitrary component(s) such as a component which is effective for the growth promotion of a ruminant, a nutritional supplement component, a component for enhancing the preservation stability, or a coating component. Examples of the arbitrary components include raw materials for a feed, a food additive, raw materials for a food and a food additive, and raw materials for a medicine. For example, the followings are included: probiotics such as *Enterococcus, Bacillus,* and *Bifidus;* enzymes such as amylase and lipase; vitamins such as L-ascrobic acid, choline chloride, inositol, and folate; minerals such as potassium chloride, iron citrate, magnesium oxide, and phosphates; amino acids such as DL-alanine, DL-methionine, and L-lysine; organic acids such as fumaric acid, butyric acid, lactic acid, acetic acid, and their salts; antioxidants such as ethoxyquin, dibutylhydroxytoluene, butylhydroxyanisol, ferulic acid, vitamin C, and vitamin E; fungicides such as calcium propionate; binders such as carboxylmethyl cellulose (CMC), casein sodium, and sodium polyacrylate; emulsifiers such as lecithin, glycerin fatty acid ester and sorbitan fatty acid ester; pigments such as astaxanthin and canthaxanthin; and flavoring agents such as various esters, ethers, and ketones.

A dosage form of the bloat therapeutic agent of the present invention is not particularly limited, and the agent may be in an arbitrary form such as a powder formulation, a liquid formulation, a solid, a tablet, a capsule, an emulsion, a pellet, and a coated formulation, and preferred are a powder formulation, a liquid formulation, a capsule, a pellet, and a coated formulation.
The powder formulation may be obtained by adding the excipient to the cashew nut shell liquid, heated cashew nut shell liquid, anacardic acid, cardanol, or anacardic acid and cardanol and forming the mixture into powder.
As the liquid formulation, the cashew nut shell liquid, heated cashew nut shell liquid, anacardic acid, cardanol, or anacardic acid and cardanol may be used as it is, or the cashew nut shell liquid, heated cashew nut shell liquid, anacardic acid, cardanol, or anacardic acid and cardanol may be used after the excipient or an arbitrary component is added thereto.
The capsule may be obtained by filling the cashew nut shell liquid, heated cashew nut shell liquid, anacardic acid, cardanol, or anacardic acid and cardanol into a capsule as it is, or by adding the excipient or an arbitrary component thereto.
The pellet may be obtained by adding the excipient to the cashew nut shell liquid, heated cashew nut shell liquid, anacardic acid, cardanol, or anacardic acid and cardanol, and granulating and pelletizing the mixture.
The coated formulation may be obtained by adding the excipient to the cashew nut shell liquid, heated cashew nut shell liquid, anacardic acid, cardanol, or anacardic acid and cardanol, granulating the mixture, and coating the resultant with a coating agent or the like.

As described above, the bloat therapeutic agent of the present invention can be produced by mixing the cashew nut shell liquid, heated cashew nut shell liquid, anacardic acid, cardanol, or anacardic acid and cardanol with, if necessary, an excipient or an arbitrary component and formulating the mixture. Note that, depending on the form of the formulation, the above-mentioned pulverized/crushed product of the cashew nut shell or the cashew nut shell as it is without being subjected to any treatment is mixed with another arbitrary component(s), and the mixture can be used as the bloat therapeutic agent of the present invention. In addition, without being mixed with another arbitrary component(s), the pulverized/crushed product as it is or the cashew nut shell as it is may be used as a bloat therapeutic agent, and the bloat therapeutic agent as it is may also be used as a composition for a feed or a feed.

The composition for a feed of the present invention includes cashew nut shell liquid, heated cashew nut shell liquid, anacardic acid, cardanol, or anacardic acid and cardanol. The content of the cashew nut shell liquid, heated cashew nut shell liquid, anacardic acid, cardanol, or anacardic acid and cardanol in the composition for a feed of the present invention is, from the viewpoints of effects and costs, preferably 0.0005 to 100 mass%, more preferably 0.05 to 90 mass%, and still more preferably 1 to 80 mass% with respect to a dry mass of the composition for a feed.
In the case where the bloat therapeutic agent of the present invention is used as a composition for a feed, the bloat therapeutic agent is mixed with another supplement component used in supplements for animals (hereinafter referred to as supplement) and may be used as a supplement. The kind of the supplement and the components other than the cashew nut shell liquid are not particularly limited.

The feed of the present invention includes cashew nut shell liquid, heated cashew nut shell liquid, anacardic acid, cardanol, or anacardic acid and cardanol.
Note that the content of the cashew nut shell liquid, heated cashew nut shell liquid, anacardic acid, cardanol, or anacardic acid and cardanol in the feed of the present invention is, from the viewpoints of effects and costs, preferably 0.5 to 500, 000 mass ppm, more preferably 5 to 300, 000 mass ppm, and still more preferably 50 to 100,000 mass ppm with respect to a dry mass of the feed.

The feed of the present invention can be produced by adding the cashew nut shell liquid, heated cashew nut shell liquid, anacardic acid, cardanol, or anacardic acid and cardanol as it is or the composition for a feed comprising the cashew nut shell liquid, heated cashew nut shell liquid, anacardic acid, cardanol, or anacardic acid and cardanol to a feed component (s) and mixing the resultant. On this occasion, when a powdery or solid composition for feed is used, the form of the composition for a feed may be modified into liquid form or gel form for the purpose of facilitating the mixing process. In this case, the following may be used as a liquid carrier: water, fluid such as a vegetable oil, a liquid animal oil, a mineral oil, a synthetic oil, and a water-soluble polymer compound. Further, in order to keep the uniformity of the cashew nut shell liquid, heated cashew nut shell liquid, anacardic acid, cardanol, or anacardic acid and cardanol in the feed, the feed preferably contains alginic acid, sodium alginate, a xanthan gum, casein sodium, an arabic rubber, a guar gum, or a water-soluble polysaccharide such as tamarind seed polysaccharide.

The species of animals that ingest the feed of the present invention is preferably ruminants. The feed of the present invention is suitable for breeding, for example, ruminants such as cows, goats, and sheep. The amount of feed ingested by an animal may be appropriately adjusted depending on the animal's species, body weight, age, sex, health condition, feed component, etc. In this case, the amount of cashew nut shell liquid, heated cashew nut shell liquid, anacardic acid, cardanol, or anacardic acid and cardanol contained in the feed is preferably 1 to 1000 ml per ruminant per day, more preferably 5 to 500 ml per ruminant per day, and still more preferably 10 to 300 ml per ruminant per day.
Any method usually used may be adopted as a method of feeding animals and a method of breeding animals depending on the species of animals.

### Examples

### Production Example

500 kg of cashew nut shells was obtained from Cashew Trading Co., Ltd., and the shells were compressed, to thereby produce 158 kg of cashew nut shell liquid. Further, heated cashew nut shell liquid, wherein anacardic acid was converted to cardanol by heat treatment at 130 °C, was obtained from Cashew Trading Co., Ltd.
The composition of cashew nut shell liquid was measured as follows. That is, HPLC (Waters600, Nihon Waters K.K.), detector (Waters490E, Nihon Waters K.K.), printer (Chromatopack C-R6A, Simadzu Co. ) , and columns (SUPELCOSIL LC18, SUPELCO Inc. ) were used. A solvent of acetonitrile: water: acetic acid in 80: 20:1 (dose ratio) was used and the flow rate was 2 ml/minute. The absorbance was detected at 280 nm.
The composition of non-heated cashew nut shell liquid was 61. 8 mass% of anacardic acid, 8.2 mass% of cardanol., and 19.9 mass% of cardol, and the composition of heated cashew nut shell liquid was 0.0 mass% of anacardic acid, 71.4 mass% of cardanol, and 14.4 mass% of cardol.

### [Example 1]

A Holstein bull, which repeated generation of chronic gas and received therapy with an anti-foaming agent about ten times and in which the symptom was not alleviated, was subjected to a test. The test bull was 6-month-old, and had a weight of about 300 kg. In the test, the day on which administration was initiated was defined as day 0. 50 ml of the cashew nut shell liquid were filled into a 60-ml injection syringe (product of TERUMO CORPORATION) to which a needle was not attached. The administration was performed by forced oral administration, and the amount of the liquid to be administered was adjusted by a scale on the injection syringe. After that, the administration was performed in accordance with the schedule shown in Table 1.

**[Table 1]**

| Day | Dose | Bloat index* |
|---|---|---|
| Day 0 | 100ml | 3 |
| Day 1 | 50ml | 1 |
| Day 2 | 40ml | 1 |
| Day 3 | 40ml | 0 |
| Day 4 | 40ml | 0 |

| | | |
|---|---|---|
| *Bloat index was evaluated into 4 grades depending on the degree of bloating (0: no bloating, 1 : mild bloating, 2: moderate bloating, 3: severe bloating) | | |

The bloating caused by gas generation was alleviated on day 1 after the administration of the cashew nut shell liquid was started, and the symptom disappeared on day 3. The bloating was cured by the administrations up to day 4, and hence, the provision of the cashew nut shell liquid was terminated. Relapse was not observed even after the elapse of 3 months. From the result, it can be considered that the cashew nut shell liquid has a therapeutic effect on bloat.

### [Example 2]

A Holstein cow, in which gas generation was once subsided by performing perforation therapy to rumen 5 days before the start of the test, but the gas generation was observed again, was subjected to a test. The test cow was 2-month-old, and had a weight of about 100 kg. In the test, the day on which administration was initiated was defined as day 0. In the same manner as in Example 1, there was performed forced oral administration of the cashew nut shell liquid. As shown in Table 2, in order to confirm an effective dose, the administration was started from a low dose and the dose was gradually increased, which was in a different manner from Example 1.

**[Table 2]**

| Day | Dose | Bloat index* |
|---|---|---|
| Day 0 | 10ml | 1 |
| Day 1 | 20ml | 2 |
| Day 2 | 30ml | 2 |
| Day 3 | 30ml | 1 |
| Day 4 | 30ml | 1 |
| Day 5 | 40ml | 1 |
| Day 6 | 40ml | 0 |
| Day 7 | 40ml | 0 |

| | | |
|---|---|---|
| *Bloat index was evaluated into 4 grades depending on the degree of bloating (0: no bloating, 1: mild bloating, 2: moderate bloating, 3: severe bloating) | | |

No remarkable alleviation of bloating was observed on the start day of the test, and hence, the dose was increased to 20 ml on day 1, and deterioration of bloating caused by gas generation was suppressed. The gas generation was subsided on day 5 by increasing the dose to 40 ml per day, and the symptom disappeared on day 6. The bloating was cured by the administrations up to day 7, and hence, the provision of the cashew nut shell liquid was terminated. Relapse was not observed even after the elapse of 3 months. From the result, it can be considered that the cashew nut shell liquid has a therapeutic effect on bloat.

### [Example 3]

A Holstein bull, which was diagnosed as chronic bloat, was subjected to a test. The test bull was 5-month-old, and had a weight of 230 kg. In the test, the day on which administration was initiated was defined as day 0. 50 ml of the heated cashew nut shell liquid were filled into a 60-ml injection syringe (product of TERUMO CORPORATION) to which a needle was not attached. The administration was performed by forced oral administration, and the amount of the liquid to be administered was adjusted by a scale on the injection syringe. After that, the administration was performed in accordance with the schedule shown in Table 3.

**[Table 3]**

| Day | Dose | Bloat index* |
|---|---|---|
| Day 0 | 100ml | 3 |
| Day 1 | 50ml | 0 |
| Day 2 | 50ml | 0 |
| Day 3 | 50ml | 0 |
| Day 4 | 50ml | 1 |
| Day 5 | 50ml | 1 |
| Day 6 | 50ml | 1 |
| Day 7 | 50ml | 0 |
| Day 8 | 50ml | 1 |
| Day 9 | 50ml | 0 |
| Day 10 | 50ml | 0 |

| | | |
|---|---|---|
| *Bloat index was evaluated into 4 grades depending on the degree of bloating (0: no bloating, 1: mild bloating, 2: moderate bloating, 3: severe bloating) | | |

It was confirmed that the bloat index of the test bull, which had repeated chronic gas generation, was remarkably and immediately alleviated by the oral administration of the heated cashew nut shell liquid. From the result, it can be considered that the heated cashew nut shell liquid has an immediate therapeutic effect on bloat.

### Industrial Applicability

By administering the cashew nut shell liquid to a ruminant sufferingfrom bloat,the bloatcan becontrolled. Further, although the ruminant may result in death in the case where the ruminant suffers from acute bloat, the bloat therapeutic agent of the present invention is also capable of controlling the acute bloat. Still further, although the therapy is expensive and good quality meat cannot be obtained due to poor growth, and therefore meat cannot be sold at a high price, in the case where the ruminant suffers from chronic bloat, relapse does not occur once the symptom is alleviated by using the bloat therapeutic agent of the present invention, and hence, the bloat therapeutic agent enables to realize cost reduction.

## Claims

1. A bloat therapeutic agent comprising cashew nut shell liquid, heated cashew nut shell liquid, anacardic acid, cardanol, or anacardic acid and cardanol.

2. A composition for a feed for bloat therapy, comprising cashew nut shell liquid, heated cashew nut shell liquid, anacardic acid, cardanol, or anacardic acid and cardanol.

3. A feed for bloat therapy, comprising cashew nut shell liquid, heated cashew nut shell liquid, anacardic acid, cardanol, or anacardic acid and cardanol.

4. The bloat therapeutic agent according to claim 1, wherein the bloat is caused by *Streptococcus bovis.*

5. A composition for a feed for bloat therapy, comprising the bloat therapeutic agent according to claim 1 or 4.

6. A feed for bloat therapy, comprising the composition for a feed for bloat therapy according to claim 5.

7. A method for bloat therapy, comprising administering cashew nut shell liquid, heated cashew nut shell liquid, anacardic acid, cardanol, or anacardic acid and cardanol to a ruminant suffering from bloat.

8. The method for bloat therapy according to claim 7, wherein the cashew nut shell liquid, heated cashew nut shell liquid, anacardic acid, cardanol, or anacardic acid and cardanol is administered 1 to 1,000 ml per ruminant per day.

9. The method for bloat therapy according to claim 7 or 8, wherein the bloat is caused by *Streptococcus bovis.*

10. Cashew nut shell liquid, heated cashew nut shell liquid, anacardic acid, cardanol, or anacardic acid and cardanol for use in manufacturing a bloat therapeutic agent.

11. Cashew nut shell liquid, heated cashew nut shell liquid, anacardic acid, cardanol, or anacardic acid and cardanol for use in manufacturing a composition for a feed for bloat therapy.

12. Cashew nut shell liquid, heated cashew nut shell liquid, anacardic acid, cardanol, or anacardic acid and cardanol for use in manufacturing a feed for bloat therapy.

13. Cashew nut shell liquid, heated cashew nut shell liquid, anacardic acid, cardanol, or anacardic acid and cardanol according to any one of claims 10 to 12, wherein the bloat is caused by *Streptococcus bovis.*
